# FASCICULE DE BREVET EUROPEEN

(11) **EP 0 565 579 B1**
(45) Date de publication et mention de la délivrance du brevet: **20.07.1994**
(21) Numéro de dépôt: 92902345.5
(22) Date de dépôt: 20.12.1991
(51) Int. Cl.: C07D 277/82, C07D 417/06, C07D 417/12, A61K 31/425, A61K 31/445

(54) **DERIVES D'IMINO-2 POLYFLUOROALKYL-6 HETEROCYCLYLALKYL-3 BENZOTHIAZOLINE, LEUR PREPARATION ET LES MEDICAMENTS LES CONTENANT**
2-IMINO-6-POLYFLUORALKYL-3-HETEROCYCLYLALKYL-BENZOTHIAZOLINDERIVATE, IHRE HERSTELLUNG UND SIE ENTHALTENDE ARZNEIMITTEL
2-IMINO 6-POLYFLUORALKYL 3-HETEROCYCLYLALKYL BENZOTHIAZOLINE DERIVATIVES, THEIR PREPARATION AND DRUGS CONTAINING SAME

(30) Priorité: 02.01.1991 FR 9100013
(43) Date de publication de la demande: 20.10.1993
(73) Titulaire: RHONE-POULENC RORER S.A., 92165 Antony Cédex (FR)
(72) Inventeur: GUEREMY, Claude, F-78800 Houilles (FR); JIMONET, Patrick, F-78450 Villepreux (FR)
(74) Mandataire: Savina, Jacques
(86) Numéro de dépôt international: FR9101041
(87) Numéro de publication internationale: WO9212142

(56) Documents cités:
- EP-A- 0 374 040

## Description

La présente invention concerne des composés de formule : 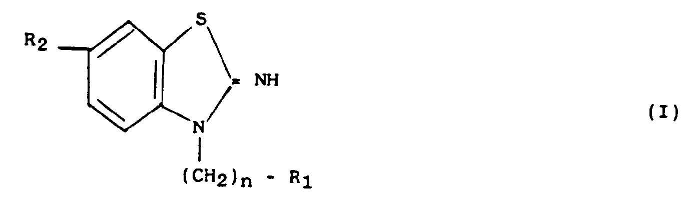
leurs sels, leurs procédés de préparation et les médicaments les contenant.
Des dérivés de benzothiazoline sont décrits dans la demande de brevet EP 374 040.
Dans la formule (I),
- R₁ représente
   . un radical pipérazinyl-1 substitué en position -4 par (a) un radical phényle, (b) un radical phényle substitué par au moins un substituant choisi parmi les atomes d'halogène, les radicaux alkyle et alcoxy, (c) un radical phénylalkyle, (d) un radical pyridyle ou (e) un radical pyrimidinyle,
   . un radical tétrahydro-1,2,3,6 pyridyl-1 substitué en position -4 par un radical phényle ou phényle substitué par au moins un substituant choisi parmi les atomes d'halogène, les radicaux alkyle et alcoxy,
   . un radical pipéridino substitué en position -4 par un radical phényle ou phényle substitué par au moins un substituant choisi parmi les atomes d'halogène, les radicaux alkyle et alcoxy,
- R₂ représente un radical polyfluoroalkyle,
- n est égal à 2 ou 3,

Dans les définitions qui précédent et celles qui seront citées ci-après, les radicaux alkyle et alcoxy et les portions alkyle et alcoxy contiennent 1 à 4 atomes de carbone en chaîne droite ou ramifiée.

Les radicaux polyfluoroalkyle sont de préférence les radicaux trifluorométhyle, trifluoro-2,2,2 éthyle, pentafluoroéthyle.

Les atomes d'halogène sont de préférence les atomes de fluor, de chlore et de brome.

L'invention concerne également les sels d'addition des composés de formule (I) avec les acides minéraux ou organiques.

Les composés de formule (I) peuvent être préparés par hydrolyse d'un dérivé de formule : 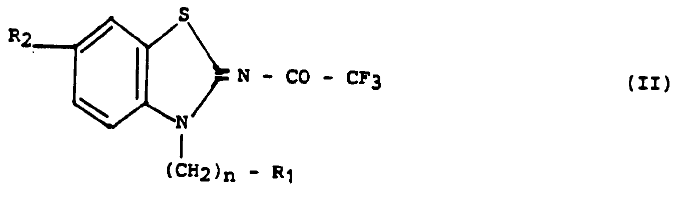
dans laquelle R₁, R₂ et n ont les mêmes significations que dans la formule (I).

Cette hydrolyse s'effectue généralement au moyen d'une base telle qu'un carbonate de métal alcalin (sodium, potassium de préférence) ou l'ammoniaque concentrée, au sein d'un mélange eau-alcool, à une température voisine de 20°C.

Les dérivés de formule (II) peuvent être obtenus par action d'un dérivé de formule : 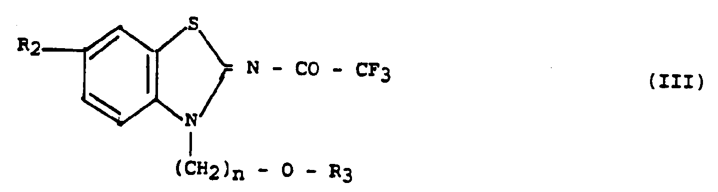
dans laquelle R₂ et n ont les mêmes significations que dans la formule (I) et R₃ représente un groupe réactif tel qu'un radical méthanesulfonyle ou p-toluènesulfonyle, sur une amine de formule :

HR₁ (IV)

dans laquelle R₁ a les mêmes significations que dans la formule (I).

Cette réaction s'effectue généralement au sein d'un solvant inerte tel qu'un solvant aromatique (benzène, toluène, xylène par exemple) ou le diméthylformamide, à une température comprise entre 20°C et la température d'ébullition du solvant.

Les dérivés de formule (III) peuvent être préparés par action d'un dérivé de formule : 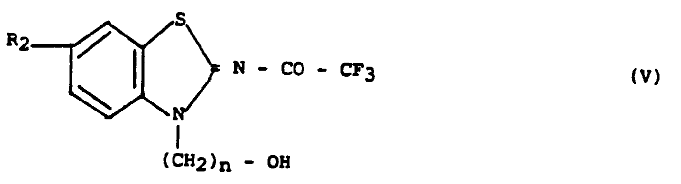
dans laquelle R₂ et n ont les mêmes significations que dans la formule (I), sur le chlorure d'acide méthanesulfonique ou p-toluènesulfonique, soit au sein d'un solvant inerte tel qu'un solvant aromatique (benzène, toluène, xylène par exemple) ou un solvant chloré (chloroforme, chlorure de méthylène par exemple), en présence d'une amine tertiaire telle que la triéthylamine, à une température voisine de 20°C, soit au sein de la pyridine, à une température voisine de 0°C.

Les dérivés de formule (V) peuvent être obtenus par action de trifluoroacétate d'éthyle sur un dérivé de formule : 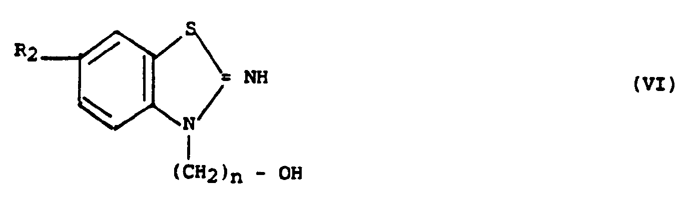
dans laquelle R₂ et n a les mêmes significations que dans la formule (I).

Cette réaction s'effectue généralement au sein d'un alcool (méthanol, éthanol par exemple), en présence d'une base tertiaire telle que la triéthylamine, à une température voisine de 20°C.

Les dérivés de formule (VI) peuvent être préparés par action d'un dérivé halogéné de formule :

Hal - (CH₂)ₙ - OH (VII)

dans laquelle n a les mêmes significations que dans la formule (I) et Hal représente un atome d'halogène (brome ou chlore de préférence) sur un amino-2 polyfluoroalkyl-6 benzothiazole.

Cette réaction s'effectue au sein d'un alcool (éthanol, méthanol de préférence), à la température d'ébullition du solvant.

Les amino-2 polyfluoroalkyl-6 benzothiazoles peuvent être préparés par application ou adaptation de la méthode décrite par L.M. YAGUPOL'SKII et coll., Zh. Obshch. Khim., 33(7), 2301, (1963).

Les composés de formule (I) peuvent également être préparés par action de brome et d'un thiocyanate de métal alcalin sur un dérivé de formule : 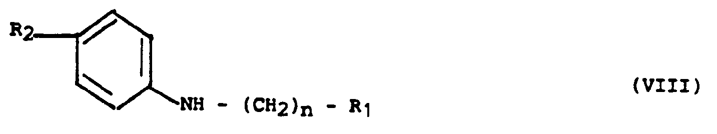
dans laquelle R₁, R₂ et n ont les mêmes significations que dans la formule (I).

Cette réaction s'effectue de préférence au sein de l'acide acétique, à une température voisine de 20°C.

Comme thiocyanate de métal alcalin, on utilise de préférence le thiocyanate de potassium.

Les dérivés de formule (VIII) peuvent être obtenus par action d'une amine de formule (IV) sur un dérivé de formule : 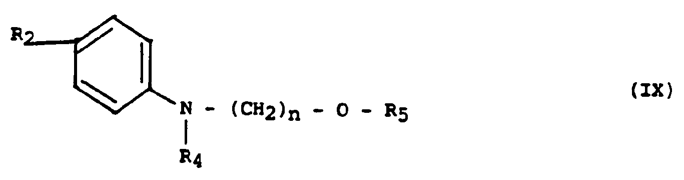
dans laquelle R₂ et n ont les mêmes significations que dans la formule (I) et R₄ et R₅ représentent un radical p-toluènesulfonyle.

Cette réaction s'effectue de préférence en présence d'hydrogénocarbonate de sodium, au sein d'un solvant inerte tel que le diméthylformamide, à une température comprise entre 50°C et 100°C.

Les dérivés de formule (IX) peuvent être obtenus par action de chlorure de p-toluènesulfonyle sur un dérivé de formule : 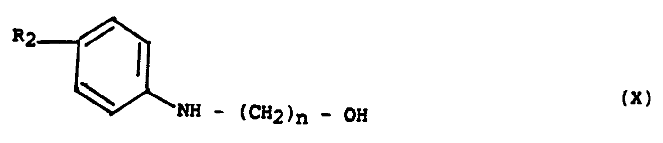
dans laquelle R₂ et n ont les mêmes significations que dans la formule (I).

Cette réaction s'effectue généralement au sein d'un solvant inerte tel qu'un solvant chloré (chloroforme, chlorure de méthylène par exemple), à une température comprise entre 0°C et 30°C.

Les dérivés de formule (X) peuvent être obtenus par action d'une polyfluoroalkyl-4 aniline sur un dérivé de formule (VII).

Cette réaction s'effectue généralement à une température comprise entre 100°C et 170°C.

Les mélanges réactionnels obtenus par les divers procédés décrits précédemment sont traités suivant des méthodes classiques physiques (évaporation, extraction, distillation, cristallisation, chromatographie...) ou chimiques (formation de sels...).

Les composés de formule (I), sous forme de base libre, peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés sont actifs vis-à-vis des convulsions induites par le glutamate et sont donc utiles dans le traitement et la prévention des phénomènes convulsifs, des troubles schizophréniques et notamment des formes déficitaires de la schizophrénie, des troubles du sommeil, des phénomènes liés à l'ischémie cérébrale ainsi que des affections neurologiques où le glutamate peut être impliqué telles que la maladie d'Alzheimer, la maladie d'Huntington, la sclérose amyotrophique latérale et l'atrophie olivopontocérébelleuse.

L'activité des composés de formule (I) vis-à-vis des convulsions induites par le glutamate a été déterminée selon une technique inspirée de celle de I.P. LAPIN, J. Neural. Transmission, vol. 54, 229-238, (1982) ; l'injection du glutamate par voie intracérébroventriculaire étant effectuée selon une technique inspirée de celle de R. CHERMAT et P. SIMON, J. Pharmacol. (Paris), vol. 6, 489-492 (1975). Leur DE₅₀ est généralement égale ou inférieure à 10 mg/kg.

Les composés de formule (I) présentent une toxicité faible. Leur DL₅₀ est supérieure à 15 mg/kg par voie I.P. chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, phénolphtalinate, méthylène-bis-β-oxynaphtoate, chlorhydrate, sulfate, nitrate et phosphate.

Les exemples suivants, donnés à titre non limitatif, montrent comment l'invention peut être mise en pratique.

### EXEMPLE 1

1,0 g de [(phényl-4 pipérazinyl-1)-2 éthyl]-3 trifluoroacétylimino-2 trifluorométhyl-6 benzothiazoline en solution dans un mélange de 5 cm3 d'une solution aqueuse de carbonate de potassium à 7% et de 50 cm3 de méthanol sont agités à une température voisine de 20°C pendant 3 heures. Le milieu réactionnel est concentré à sec sous pression réduite (20 mm de mercure ; 2,7 kPa) et le résidu repris par de l'éther diéthylique. L'insoluble est filtré et le filtrat concentré à sec sous pression réduite. Après purification par chromatographie sur silice avec de l'acétate d'éthyle comme éluant puis formation du dichlorhydrate à l'aide d'éther chlorhydrique, on obtient 0,5 g de dichlorhydrate d'imino-2 [(phényl-4 pipérazinyl-1)-2 éthyl]-3 trifluorométhyl-6 benzothiazoline fondant à 260°C.

La [(phényl-4 pipérazinyl-1)-2 éthyl]-3 trifluoroacétylimino-2 trifluorométhyl-6 benzothiazoline peut être préparée selon le procédé suivant: à 3,5 g de p-toluènesulfonate de (trifluoroacétylimino-2 trifluorométhyl-6 benzothiazolinyl-3)-2 éthyle en solution dans 50 cm3 de diméthylformamide, on ajoute 0,6 g d'hydrogénocarbonate de sodium puis 1,2 g de N-phényl pipérazine solubilisés dans 10 cm3 de diméthylformamide. Après agitation à 50°C pendant 20 heures, le milieu réactionnel est concentré à sec sous pression réduite (7 mm de mercure ; 0,95 kPa). Le résidu pâteux est repris par 50 cm3 de dichlorométhane, l'insoluble filtré et le filtrat concentré à sec sous pression réduite (20 mm de mercure ; 2,7 kPa). Après purification par chromatographie sur colonne de silice avec un mélange acétate d'éthyle-cyclohexane (30-70 en volumes) comme éluant, on obtient 1,0g de [(phényl-4 pipérazinyl-1)-2 éthyl]-3 trifluoroacétylimino-2 trifluorométhyl-6 benzothiazoline sous forme d'une huile incolore.

Le p-toluènesulfonate de (trifluoroacétylimino-2 trifluorométhyl-6 benzothiazolinyl-3)-2 éthyle peut être préparé selon le procédé suivant: à 5,3 g de (trifluoroacétylimino-2 trifluorométhyl-6 benzothiazolinyl-3)-2 éthanol en suspension dans 40 cm3 de pyridine sèche, sous azote et à une température voisine de 0°C, on ajoute en environ 30 minutes, 5,7 g de chlorure de p-toluènesulfonyle en solution dans 10 cm3 du même solvant. La réaction est poursuivie 2 heures à une température voisine de 20°C. Le milieu réactionnel est ajouté à de l'eau glacée et acidifié à l'aide d'acide chlorhydrique 5N jusqu'à un pH voisin de 2. Le précipité blanc formé est filtré, lavé à l'eau jusqu'à neutralité puis séché à 70°C sous vide partiel (1mm de mercure ; 0,14 kPa ). On obtient ainsi 7,2 g de p-toluènesulfonate de (trifluoroacétylimino-2 trifluorométhyl-6 benzothiazolinyl-3)-2 éthyle fondant à 163°C, se reprenant pour fondre à nouveau à 215°C.

Le (trifluoroacétylimino-2 trifluorométhyl-6 benzothiazolinyl-3)-2 éthanol peut être préparé de la manière suivante: à 10,5 g de bromhydrate d'(imino-2 trifluorométhyl-6 benzothiazolinyl-3)-2 éthanol en solution dans 50 cm3 d'éthanol absolu, on ajoute 9,5 cm3 de triéthylamine puis 5,2 g de trifluoroacétate d'éthyle. Le milieu réactionnel est agité 24 heures à une température voisine de 20°C. Après concentration à sec sous pression réduite (20 mm de mercure ; 2,7 kPa), le solide obtenu est recristallisé dans un mélange éthanol-eau (70-30 en volumes). On obtient 6,55 g de (trifluoroacétylimino-2 trifluorométhyl-6 benzothiazolinyl-3)-2 éthanol fondant à 198°C.

Le bromhydrate d'(imino-2 trifluorométhyl-6 benzothiazolinyl-3)-2 éthanol peut être obtenu de la façon suivante: 17,9 g d'amino-2 trifluorométhyl-6 benzothiazole et 41,0 g de bromo-2 éthanol dans 50 cm3 d'éthanol absolu sont chauffés pendant 40 heures à ébullition. Le mélange est ensuite refroidi à une température voisine de 20°C et concentré à sec sous pression réduite (20 mm de mercure ; 2,7 kPa). Le résidu obtenu est repris dans l'éther diéthylique et l'insoluble filtré sur verre fritté, lavé abondamment avec le même solvant et séché pour conduire à 10,5 g de bromhydrate d'(imino-2 trifluorométhyl-6 benzothiazolinyl-3)-2 éthanol.

L'amino-2 trifluorométhyl-6 benzothiazole peut être préparé selon le procédé décrit dans le brevet US 2 822 359.

### EXEMPLE 2

En opérant comme à l'exemple 1 mais à partir de 3,5 g de {[(fluoro-4 phényl)-4 pipérazinyl-1]-2 éthyl}-3 trifluoroacétylimino-2 trifluorométhyl-6 benzothiazoline, de 15 cm3 d'une solution aqueuse de carbonate de potassium à 7% et de 150 cm3 de méthanol, on obtient 2,2 g de trichlorhydrate d'imino-2 {[(fluoro-4 phényl)-4 pipérazinyl-1]-2 éthyl}-3 trifluorométhyl-6 benzothiazoline fondant à 250°C.

La {[(fluoro-4 phényl)-4 pipérazinyl-1]-2 éthyl}-3 trifluoroacétylimino-2 trifluorométhyl-6 benzothiazoline peut être préparée de la façon suivante: on opère comme à l'exemple 1, à partir de 8,7 g de p-toluènesulfonate de (trifluoroacétylimino-2 trifluorométhyl-6 benzothiazolinyl-3)-2 éthyle, de 3,2 g de N-(fluoro-4 phényl)-pipérazine, de 1,43 g d'hydrogénocarbonate de sodium et de 125 cm3 de diméthylformamide. On obtient 3,5 g de produit attendu sous forme d'un solide jaune pâteux utilisé dans les synthèses ultérieures.

### EXEMPLE 3

En opérant comme à l'exemple 1 mais à partir de 0,1 g de {[(méthoxy-4 phényl)-4 pipérazinyl-1]-2 éthyl}-3 trifluoroacétylimino-2 trifluorométhyl-6 benzothiazoline, de 0,5 cm3 d'une solution aqueuse de carbonate de potassium à 7% et de 5 cm3 de méthanol, on obtient 0,095 g de dichlorhydrate d'imino-2 {[(méthoxy-4 phényl)-4 pipérazinyl-1]-2 éthyl}-3 trifluorométhyl-6 benzothiazoline fondant à environ 270°C.

La {[(méthoxy-4 phényl)-4 pipérazinyl-1]-2 éthyl}-3 trifluoroacétylimino-2 trifluorométhyl-6 benzothiazoline peut être préparée de la manière suivante: on opère comme à l'exemple 1, à partir de 3,5 g de p-toluènesulfonate de (trifluoroacétylimino-2 trifluorométhyl-6 benzothiazolinyl-3)-2 éthyle, de 1,9 g de dichlorhydrate de N-(méthoxy-4 phényl)-pipérazine, de 1,2 g d'hydrogénocarbonate de sodium et de 60 cm3 de diméthylformamide. On obtient 0,1 g de produit attendu sous forme de solide blanchâtre amorphe fondant à 120°C.

La présente invention concerne également les médicaments constitués par au moins un composé de formule (I) ou un sel d'un tel composé à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, pilules, poudres (capsules de gélatine, cachets ou granulés. Dans ces compositions, le principe actif selon l'invention est mélangé à un ou plusieurs diluants inertes, tels que amidon, cellulose, saccharose, lactose ou silice.

Ces composition peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectables qui contiennent, outre le produit actif, des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles dans le traitement et la prévention des phénomènes convulsifs, des troubles schizophréniques et notamment des formes déficitaires de la schizophrénie, des troubles du sommeil, des phénomènes liés à l'ischémie cérébrale et des affections neurologiques où le glutamate peut être impliqué telles que la maladie d'Alzheimer, la maladie d'Huntington, la sclérose amyotrophique latérale et l'atrophie olivopontocérébelleuse.

Les doses dépendent de l'effet recherché, de la durée du traitement et de la voie d'administration utilisée ; elles sont généralement comprises entre 30 et 300mg par jour par voie orale pour un adulte avec des doses unitaires allant de 10 à 100mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- imino-2 [(phényl-4 pipérazinyl-1)-2 éthyl]-3 trifluorométhyl-6 benzothiazoline 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare, selon la technique habituelle, des comprimés dosés à 50 mg de produit actif ayant la composition habituelle :
- imino-2 {[(fluoro-4 phényl)-4 pipérazinyl-1]-2 éthyl}-3 trifluorométhyl-6 benzothiazoline 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (71-3,5-24,5) q.s.p. 1 comprimé pelliculé terminé à 245 mg

### EXEMPLE C

On prépare une solution injectable contenant 10 mg de produit actif ayant la composition suivante :
- imino-2 {[(méthoxy-4 phényl)-4 pipérazinyl-1]-2 éthyl}-3 trifluorométhyl-6 benzothiazoline 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm³

## Revendications (Revendications pour l'(les) Etat(s) contractant(s) suivant(s): AT, BE, CH, DE, DK, FR, GB, IT, LI, LU, NL, SE)

1. Composés de formule : 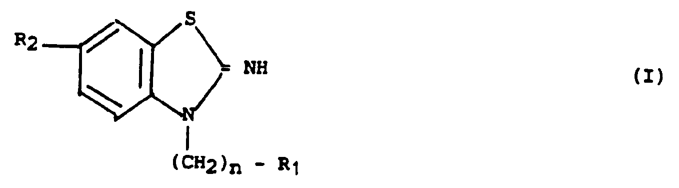 dans laquelle
- R₁ représente
. un radical pipérazinyl-1 substitué en position -4 par (a) un radical phényle, (b) un radical phényle substitué par au moins un substituant choisi parmi les atomes d'halogène, les radicaux alkyle et alcoxy, (c) un radical phénylalkyle, (d) un radical pyridyle ou (e) un radical pyrimidinyle,
. un radical tétrahydro-1,2,3,6 pyridyl-1 substitué en position -4 par un radical phényle ou phényle substitué par au moins un substituant choisi parmi les atomes d'halogène, les radicaux alkyle et alcoxy,
. un radical pipéridino substitué en position -4 par un radical phényle ou phényle substitué par au moins un substituant choisi parmi les atomes d'halogène, les radicaux alkyle et alcoxy,
- R₂ représente un radical polyfluoroalkyle,
- n est égal à 2 ou 3,
étant entendu que les radicaux alkyle et alcoxy et les portions alkyle et alcoxy contiennent 1 à 4 atomes de carbone en chaîne droite ou ramifiée,
ainsi que leurs sels avec un acide minéral ou organique.

2. Composés de formule (I) selon la revendication 1 pour lesquels R₂ représente un radical trifluorométhyle, trifluoro-2,2,2 éthyle ou pentafluoroéthyle,

3. Procédé de préparation des composés de formule (I) selon la revendication 1 caractérisé en ce que l'on hydrolyse un dérivé de formule : 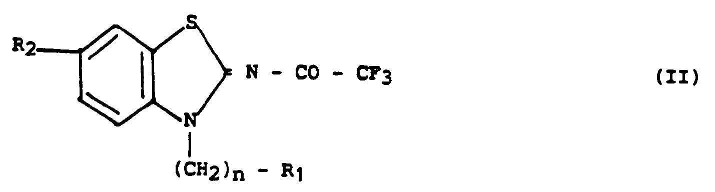 dans laquelle R₁ , R₂ et n ont les mêmes significations que dans la revendication 1, isole le produit et le transforme éventuellement en sel d'addition avec un acide minéral ou organique.

4. Procédé de préparation des composés de formule (I) selon la revendication 1 caractérisé en ce que l'on fait réagir du brome et un thiocyanate de métal alcalin sur un dérivé de formule : 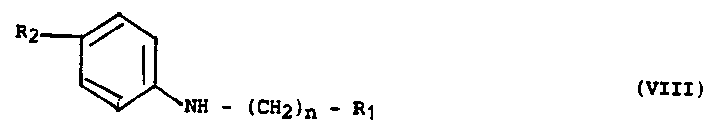 dans laquelle R₁, R₂ et n ont les mêmes significations que dans la formule (I), isole le produit et le transforme éventuellement en sel d'addition avec un acide minéral ou organique.

5. Médicaments caractérisés en ce qu'ils contiennent en tant que principe actif au moins un composé selon la revendication 1 ou un sel d'un tel composé.

6. Médicaments caractérisés en ce qu'ils contiennent en tant que principe actif au moins un composé selon la revendication 2 ou un sel d'un tel composé.

7. Médicaments selon la revendication 5 et 6 pour le traitement des affections où le glutamate est impliqué.

## Revendications (Revendications pour l'(les) Etat(s) contractant(s) suivant(s): ES, GR)

1. Procédé de préparation des composés de formule : 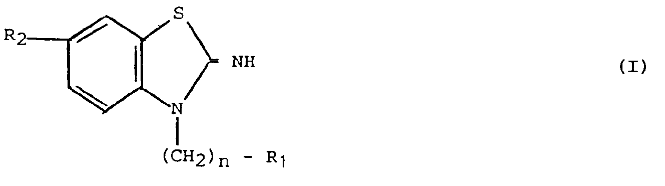 dans laquelle
- R₁ représente
. un radical pipérazinyl-1 substitué en position -4 par (a) un radical phényle, (b) un radical phényle substitué par au moins un substituant choisi parmi les atomes d'halogène, les radicaux alkyle et alcoxy, (c) un radical phénylalkyle, (d) un radical pyridyle ou (e) un radical pyrimidinyle,
. un radical tétrahydro-1,2,3,6 pyridyl-1 substitué en position -4 par un radical phényle ou phényle substitué par au moins un substituant choisi parmi les atomes d'halogène, les radicaux alkyle et alcoxy,
. un radical pipéridino substitué en position -4 par un radical phényle ou phényle substitué par au moins un substituant choisi parmi les atomes d'halogène, les radicaux alkyle et alcoxy,
- R₂ représente un radical polyfluoroalkyle,
- n est égal à 2 ou 3,
étant entendu que les radicaux alkyle et alcoxy et les portions alkyle et alcoxy contiennent 1 à 4 atomes de carbone en chaîne droite ou ramifiée,
ainsi que leurs sels avec un acide minéral ou organique,
caractérisé en ce que :
A - on hydrolyse un dérivé de formule : 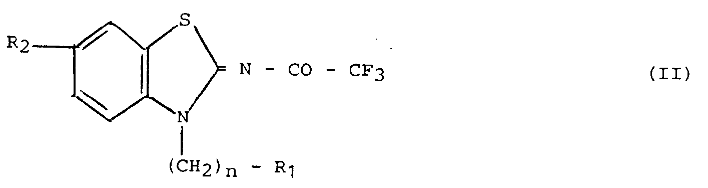 dans laquelle R₁ , R₂ et n ont les mêmes significations que dans la formule (I), isole le produit et le transforme éventuellement en sel d'addition avec un acide minéral ou organique, ou
B - on fait réagir du brome et un thiocyanate de métal alcalin sur un dérivé de formule : 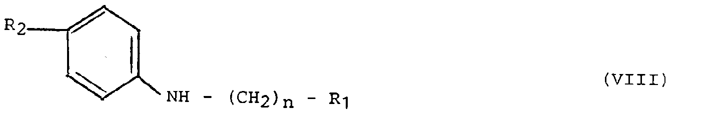 dans laquelle R₁, R₂ et n ont les mêmes significations que dans la formule (I), isole le produit et le transforme éventuellement en sel d'addition avec un acide minéral ou organique.

2. Procédé selon la revendication 1 pour la préparation des composés de formule (I) pour lesquels R₂ représente un radical trifluorométhyle, trifluoro-2,2,2 éthyle ou pentafluoroéthyle.

## Patentansprüche (Patentansprüche für folgende(n) Vertragsstaat(en): AT, BE, CH, DE, DK, FR, GB, IT, LI, LU, NL, SE)

1. Verbindungen der Formel 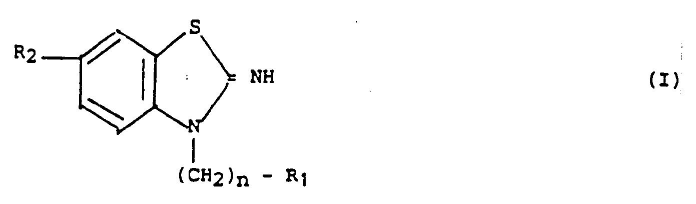 worin
- R₁ bedeutet:
. einen Piperazin-1-ylrest, der substituiert ist in 4-Stellung durch (a) einen Phenylrest, (b) einen Phenylrest, substituiert durch zumindest einen Substituenten, ausgewählt unter den Halogenatomen, den Alkyl- und Alkoxyresten, (c) einen Phenylalkylrest, (d) einen Pyridylrest oder (e) einen Pyrimidinylrest,
. einen 1,2,3,6-Tetrahydropyrid-1-ylrest, der substituiert ist in 4-Stellung durch einen Phenylrest oder durch einen Phenylrest, substituiert durch zumindest einen Substituenten, ausgewählt unter den Halogenatomen, den Alkyl- und Alkoxyresten,
. einen Piperidinorest, der substituiert ist in 4-Stellung durch einen Phenylrest oder durch einen Phenylrest, substituiert durch zumindest einen Substituenten, ausgewählt unter den Halogenatomen, den Alkyl- und Alkoxyresten,
- R₂ einen Polyfluoralkylrest bedeutet,
- n für 2 oder 3 steht,
mit der Maßgabe, daß die Alkyl- und Alkoxyreste und die Alkyl- und Alkoxyteile 1 bis 4 Kohlenstoffatome in gerader oder verzweigter Kette aufweisen,
sowie deren Salze mit einer Mineral- oder organischen Säure.

2. Verbindungen der Formel (I) gemäß Anspruch 1, worin R₂ einen Trifluormethyl-, 2,2,2-Trifluorethyl- oder Pentafluorethylrest bedeutet.

3. Verfahren zur Herstellung der Verbindungen der Formel (I) gemäß Anspruch 1, dadurch gekennzeichnet, daß man ein Derivat der Formel 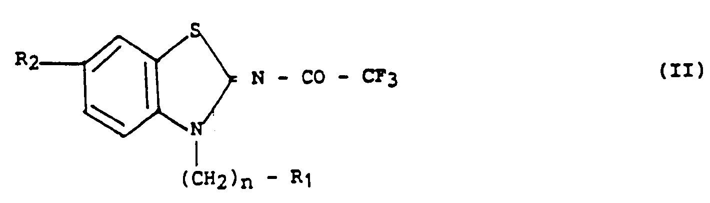 worin R₁, R₂ und n die in Anspruch 1 angegebenen Bedeutungen besitzen, hydrolysiert, das Produkt isoliert und es gegebenenfalls in ein Additionssalz mit einer Mineral- oder organischen Säure überführt.

4. Verfahren zur Herstellung der Verbindungen der Formel (I) gemäß Anspruch 1, dadurch gekennzeichnet, daß man Brom und ein Alkalimetallthiocyanat mit einem Derivat der Formel 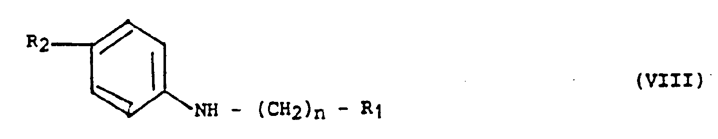 umsetzt, worin R₁, R₂ und n die für Formel (I) angegebenen Bedeutungen besitzen, das Produkt isoliert und es gegebenenfalls in ein Additionssalz mit einer Mineral- oder organischen Säure überführt.

5. Arzneimittel, dadurch gekennzeichnet, daß sie als Wirkstoff zumindest eine Verbindung gemäß Anspruch 1 oder ein Salz einer derartigen Verbindung enthalten.

6. Arzneimittel, dadurch gekennzeichnet, daß sie als Wirkstoff zumindest eine Verbindung gemäß Anspruch 2 oder ein Salz einer derartigen Verbindung enthalten.

7. Arzneimittel gemäß den Ansprüchen 5 und 6 für die Behandlung von Erkrankungen, bei denen das Glutamat impliziert ist.

## Patentansprüche (Patentansprüche für folgende(n) Vertragsstaat(en): ES, GR)

1. Verfahren zur Herstellung der Verbindungen der Formel (I) 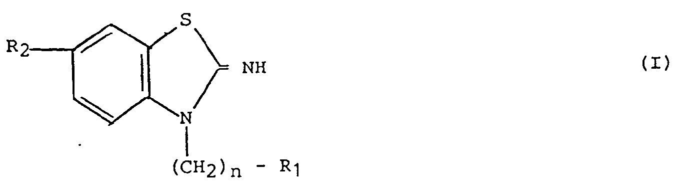 worin
- R₁ bedeutet:
. einen Piperazin-1-ylrest, der substituiert ist in 4-Stellung durch (a) einen Phenylrest, (b) einen Phenylrest, substituiert durch zumindest einen Substituenten, ausgewählt unter den Halogenatomen, den Alkyl- und Alkoxyresten, (c) einen Phenylalkylrest, (d) einen Pyridylrest oder (e) einen Pyrimidinylrest,
. einen 1,2,3,6-Tetrahydropyrid-1-ylrest, der substituiert ist in 4-Stellung durch einen Phenylrest oder durch einen Phenylrest, substituiert durch zumindest einen Substituenten, ausgewählt unter den Halogenatomen, den Alkyl- und Alkoxyresten,
. einen Piperidinorest, der substituiert ist in 4-Stellung durch einen Phenylrest oder durch einen Phenylrest, substituiert durch zumindest einen Substituenten, ausgewählt unter den Halogenatomen, den Alkyl- und Alkoxyresten,
- R₂ einen Polyfluoralkylrest bedeutet,
- n für 2 oder 3 steht,
mit der Maβgabe, daβ die Alkyl- und Alkoxyreste und die Alkyl- und Alkoxyteile 1 bis 4 Kohlenstoffatome in gerader oder verzweigter Kette aufweisen,
sowie von deren Salzen mit einer Mineral- oder organischen Säure, dadurch gekennzeichnet, daß man
A - ein Derivat der Formel 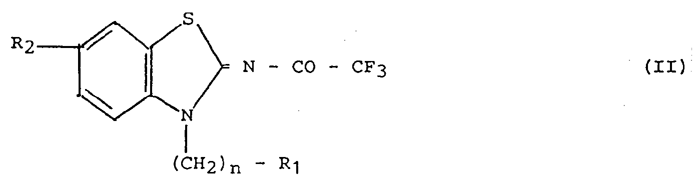 hydrolysiert, worin R₁, R₂ und n die fur Formel (I) angegebenen Bedeutungen besitzen, das Produkt isoliert und es gegebenenfalls in ein Additionssalz mit einer Mineral- oder organischen Säure überführt, oder
B- Brom und ein Alkalimetallthiocyanat mit einem Derivat der Formel 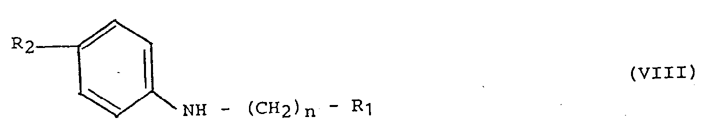 umsetzt, worin R₁, R₂ und n die für Formel (I) angegebenen Bedeutungen besitzen, das Produkt isoliert und es gegebenenfalls in ein Additionssalz mit einer Mineral- oder organischen Säure überführt.

2. Verfahren gemäβ Anspruch 1 zur Herstellung der Verbindungen der Formel (I), worin R₂ einen Trifluormethyl-, 2,2,2-Trifluorethyl- oder Pentafluorethylrest bedeutet.

## Claims (Claims for the following Contracting State(s): AT, BE, CH, DE, DK, FR, GB, IT, LI, LU, NL, SE)

1. Compounds of formula: 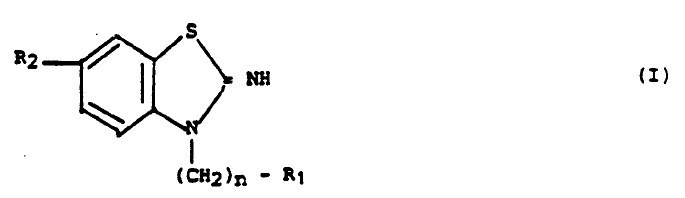 in which
- R₁ represents
. a 1-piperazinyl radical substituted in position 4 by (a) a phenyl radical, (b) a phenyl radical substituted by at least one substituent chosen from halogen atoms, alkyl and alkoxy radicals, (c) a phenylalkyl radical, (d) a pyridyl radical or (e) a pyrimidinyl radical,
. a 1,2,3,6-tetrahydro-1-pyridyl radical substituted in position 4 by a phenyl radical or a phenyl radical substituted by at least one substituent chosen from halogen atoms and alkyl and alkoxy radicals,
. a piperidino radical substituted in position 4 by a phenyl radical or a phenyl radical substituted by at least one substituent chosen from halogen atoms and alkyl and alkoxy radicals,
- R₂ represents a polyfluoroalkyl radical,
- n is equal to 2 or 3,
it being understood that the alkyl and alkoxy radicals and the alkyl and alkoxy portions contain 1 to 4 carbon atoms in a linear or branched chain,
as well as their salts with an inorganic or organic acid.

2. Compounds of formula (I) according to Claim 1, for which R₂ represents a trifluoromethyl, 2,2,2-trifluoroethyl or pentafluoroethyl radical.

3. Process for the preparation of the compounds of formula (I) according to Claim 1, characterized in that a derivative of formula: 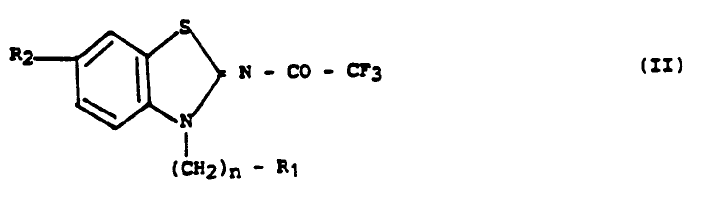 in which R₁, R₂ and n have the same meanings as in Claim 1, is hydrolysed, the product is isolated and it is optionally converted to an addition salt with an inorganic or organic acid.

4. Process for the preparation of the compounds of formula (I) according to Claim 1, characterized in that bromine and an alkali metal thiocyanate are reacted with a derivative of formula: 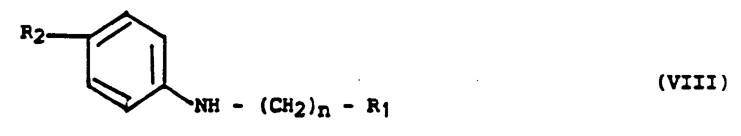 in which R₁, R₂ and n have the same meanings as in formula (I), the product is isolated and it is optionally converted to an addition salt with an inorganic or organic acid.

5. Medicinal products characterized in that they contain, as active ingredient, at least one compound according to Claim 1 or a salt of such a compound.

6. Medicinal products characterized in that they contain, as active ingredient, at least one compound according to Claim 2 or a salt of such a compound.

7. Medicinal products according to Claims 5 and 6, for the treatment of conditions where glutamate is involved.

## Claims (Claims for the following Contracting State(s): ES, GR)

1. Process for the preparation of the compounds of formula: 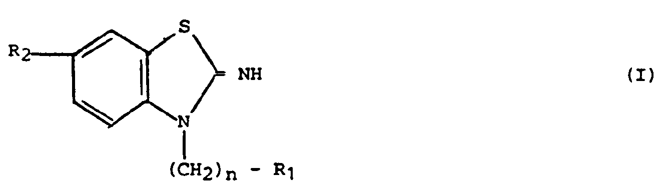 in which
- R₁ represents
. a 1-piperazinyl radical substituted in position 4 by (a) a phenyl radical, (b) a phenyl radical substituted by at least one substituent chosen from halogen atoms, alkyl and alkoxy radicals, (c) a phenylalkyl radical, (d) a pyridyl radical or (e) a pyrimidinyl radical,
. a 1,2,3,6-tetrahydro-1-pyridyl radical substituted in position 4 by a phenyl radical or a phenyl radical substituted by at least one substituent chosen from halogen atoms and alkyl and alkoxy radicals,
. a piperidino radical substituted in position 4 by a phenyl radical or a phenyl radical substituted by at least one substituent chosen from halogen atoms and alkyl and alkoxy radicals,
- R₂ represents a polyfluoroalkyl radical,
- n is equal to 2 or 3,
it being understood that the alkyl and alkoxy radicals and the alkyl and alkoxy portions contain 1 to 4 carbon atoms in a linear or branched chain,
as well as their salts with an inorganic or organic acid, characterized in that:
A - a derivative of formula: 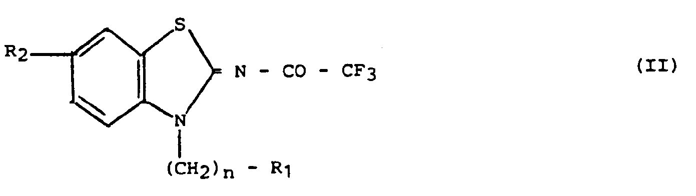 in which R₁, R₂ and n have the same meanings as in formula (I), is hydrolysed, the product is isolated and it is optionally converted to an addition salt with an inorganic or organic acid, or
B - bromine and an alkali metal thiocyanate are reacted with a derivative of formula: 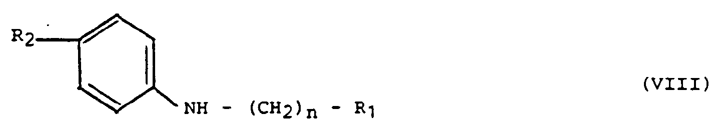 in which R₁, R₂ and n have the same meanings as in formula (I), the product is isolated and it is optionally converted to an addition salt with an inorganic or organic acid.

2. Process according to Claim 1, for the preparation of the compounds of formula (I) for which R₂ represents a trifluoromethyl, 2,2,2-trifluoroethyl or pentafluoroethyl radical.
